(19) **Europäisches Patentamt / European Patent Office / Office européen des brevets**

(11) **EP 3 634 338 B1**

(12) **EUROPEAN PATENT SPECIFICATION**

(45) Date of publication and mention
of the grant of the patent:
**27.07.2022   Bulletin 2022/30**

(21) Application number: **18734690.3**

(22) Date of filing: **04.06.2018**

(51) International Patent Classification (IPC):
***A61F 13/00*** *(2006.01)*

(52) Cooperative Patent Classification (CPC):
**A61F 13/00004; A61F 13/00017; A61F 13/00029; A61F 13/00042; A61F 13/00046**

(86) International application number:
**PCT/US2018/035873**

(87) International publication number:
**WO 2018/226592 (13.12.2018 Gazette 2018/50)**

(54) **WOUND DRESSING WITH ODOR ABSORPTION AND INCREASED MOISTURE VAPOR TRANSMISSION**

WUNDVERBAND MIT GERUCHSABSORPTION UND ERHÖHTER
FEUCHTIGKEITSDAMPFDURCHLÄSSIGKEIT

PANSEMENT POUR PLAIE AVEC ABSORPTION D'ODEURS ET TRANSMISSION ACCRUE DE
VAPEUR D'HUMIDITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **07.06.2017   US 201762516289 P**

(43) Date of publication of application:
**15.04.2020   Bulletin 2020/16**

(73) Proprietors:
• **KCI USA, Inc.**
  **San Antonio, TX 78265 (US)**
• **Systagenix Wound Management, Limited**
  **Beehive Ring Road,**
  **Gatwick Airport,**
  **West Sussex RH6 0PA (GB)**

(72) Inventors:
• **HILL, Clinton Ross**
  **Colne**
  **Lancashire BB8 9BZ (GB)**
• **POOLE, Leanna Jade**
  **Rawtenstall**
  **Lancashire BB4 8JA (GB)**
• **BOLTON, Rachel Emma**
  **Halifax HX3 6JQ (GB)**

(74) Representative: **Simmons & Simmons**
  **City Point**
  **One Ropemaker Street**
  **London EC2Y 9SS (GB)**

(56) References cited:
**WO-A1-86/05970          WO-A1-2012/008903
WO-A2-2013/007973     GB-A- 2 531 345
US-A1- 2007 293 799**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### BACKGROUND

The present disclosure relates generally to a wound dressing. The present disclosure relates more particularly to a wound dressing having an absorbent structure for the maintenance of a suitable moisture level at the surface of wounds and a reduction of odor.

Maintaining a moist wound environment can promote the healing of wounds, especially burns and chronic wounds such as ulcers. However, excessive moisture or pooling of wound exudate on the wound can cause maceration of skin adjacent to the wound and other difficulties. Furthermore, liquid exudate can leak from the wound site and contaminate clothes or bedding. It can be difficult to maintain the desired moisture level at the wound site because the rate of wound fluid production varies from wound to wound, and over time for any single wound. This can necessitate frequent dressing changes and a variety of dressing types to treat different wounds.

WO 2013/007973 A2 concerns a wound dressing including an absorbent layer and an obscuring layer. GB 2531345 A concerns a composite wound dressing comprising a superabsorbent material and a layer of active carbon cloth. WO 86/05970 concerns an integral antibacterial wound dressing comprising a first layer of permeable material, a second layer of semi-permeable material, a layer of charcoal fabric and a second layer of permeable material.

Some wound dressings are designed to promote moisture vapor transmission through the wound dressing while maintaining a moist wound environment. It is often desirable to use a wound dressing with an odor-absorbing layer. However, the moisture vapor transmission rate (MVTR) provided by a wound dressing typically decreases if additional layers are added to the wound dressing and/or if existing layers of the wound dressing are made thicker. This is because additional layers and/or thicker layers are known to obstruct moisture vapor transmission through the wound dressing and therefore have the effect of decreasing MVTR. It would be desirable to provide a wound dressing that overcomes these and other limitations of conventional wound dressings.

### SUMMARY

The present invention provides a wound dressing according to claim 1. Further optional features are provided by the dependent claims.

Those skilled in the art will appreciate that the summary is illustrative only and is not intended to be in any way limiting. Other aspects, inventive features, and advantages of the devices and/or processes described herein, as defined solely by the claims, will become apparent in the detailed description set forth herein and taken in conjunction with the accompanying drawings.

## BRIEF DESCRIPTION OF THE DRAWINGS

FIG. 1 is a top view of a wound dressing, according to an exemplary embodiment.

FIG. 2 is a bottom view of the wound dressing of FIG. 1, according to an exemplary embodiment.

FIG. 3 is an exploded view illustrating several layers of the wound dressing of FIG.1, according to an exemplary embodiment.

FIG. 4 is a cross-sectional view of the wound dressing of FIG. 1 adhered to a surface, according to an exemplary embodiment.

FIG. 5A is a data table indicating the moisture vapor transmission rate (MVTR), absorbency, and total fluid handling capacity (TFHC) of several samples of wound dressings without a charcoal layer, according to an exemplary embodiment.

FIG. 5B is a column graph plotting the test results shown in FIG. 5A, according to an exemplary embodiment.

FIG. 6A is a data table indicating the MVTR, absorbency, and TFHC of several samples of the wound dressing of FIGS. 1-4 with a charcoal layer, according to an exemplary embodiment.

FIG. 6B is a column graph plotting the test results shown in FIG. 6B , according to an exemplary embodiment.

FIG. 7A is a data table summarizing the mean test results shown in FIGS. 5A and 6A , according to an exemplary embodiment.

FIG. 7B is a column graph plotting the test results shown in FIG. 7B, according to an exemplary embodiment.

### DETAILED DESCRIPTION

#### Overview

Referring generally to the FIGURES, a wound dressing with odor absorption and increased moisture vapor transmission is shown, according to an exemplary embodiment. The wound dressing has multiple layers including a backing layer, a charcoal layer, and a superabsorbent layer laminated to a hydrophilic foam layer. In some embodiments, the superabsorbent layer and the hydrophilic foam layer are bonded to each other (e.g., laminated together using a fusible fiber) or otherwise combined to form an island.

Advantageously, the charcoal layer increases the moisture vapor transmission rate (MVTR), the absorbency, the total fluid handling capacity (TFHC) of the wound dressing relative to similar wound dressings without the charcoal layer. Such an increase in MVTR is unexpected given that adding another layer typically decreases MVTR. However, the unique arrangement and composition of layers included in the wound dressing has been shown to increase MVTR, absorbency, and TFHC.

In some embodiments, the charcoal layer includes activated charcoal (e.g., an activated charcoal cloth) having a plurality of small, low-volume pores within the internal volume of charcoal layer 104. The pores increase the internal surface area of the charcoal layer available for adsorption of the wound fluid. It is believed that the pores within the charcoal layer contribute to the unexpected increase in MVTR.

Another advantage provided by the charcoal layer is odor absorption. The charcoal layer may have odor-absorbent properties and may function to reduce wound odor. The odor-absorbent properties of the charcoal layer may result from the charcoal layer trapping odor within the plurality of small, low-volume pores. The odor-absorbent properties of the charcoal layer, in combination with the increase in MVTR, absorbency, and TFHC, provide a multifunctional wound dressing that both manages wound exudate and reduces wound odor without requiring multiple wound dressings. Additional features and advantages of the wound dressing are described in detail below.

**Wound Dressing**

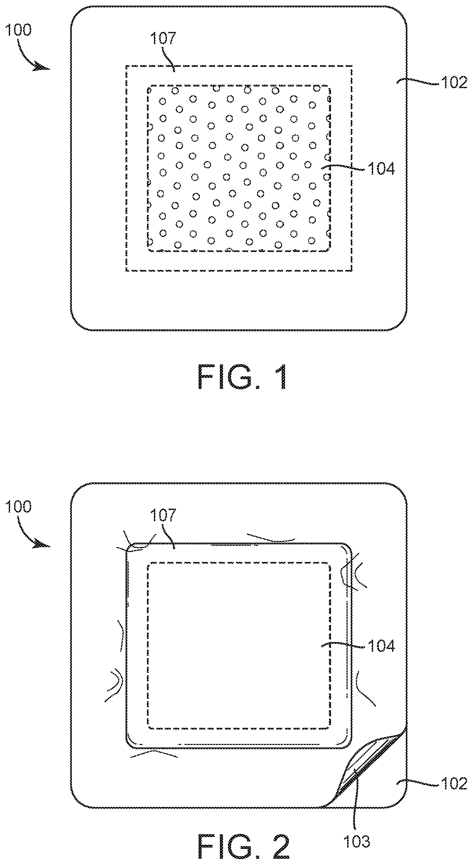

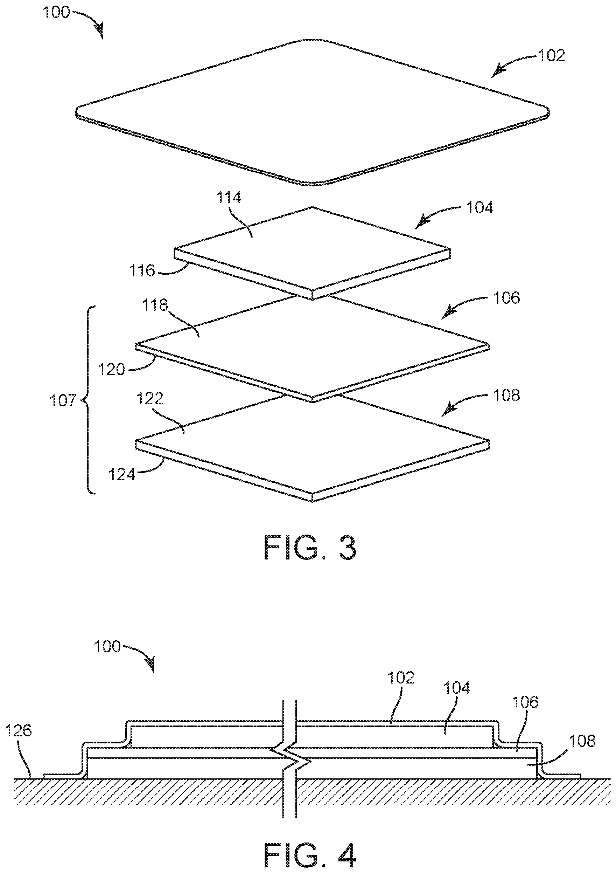

Referring now FIGS. 1-4, a wound dressing 100 is shown, according to an exemplary embodiment. In brief overview, FIG. 1 is a top view of wound dressing 100 as would be visible when wound dressing 100 is adhered to a surface (e.g., a patient's skin). FIG. 2 is a bottom view of wound dressing 100 showing the wound-contacting surface of wound dressing 100. The broken lines in FIGS. 1-2 outline the layers of wound dressing 100 that are not visible in each view. FIG. 3 is an exploded view illustrating several layers 102-108 of wound dressing 100. FIG. 4 is a cross-sectional view of wound dressing 100 adhered to a surface 126.

In various embodiments, wound dressing 100 can be formed as a substantially flat sheet for topical application to wounds or contoured for application to body surfaces having high curvature. The size of wound dressing 100 can vary depending on the size of the wound to be dressed. For example, it is contemplated that the size of wound dressing 100 can range from 1 cm² to 200 cm², and more preferably from 4 cm² to 100 cm². However, other shapes and sizes of wound dressing 100 are also possible depending on the intended use.

Wound dressing 100 is shown to include a backing layer 102, a charcoal layer 104, a superabsorbent layer 106, and a hydrophilic foam layer 108. In some embodiments, superabsorbent layer 106 and hydrophilic foam layer 108 are bonded to each other (e.g., laminated together using a fusible fiber) or otherwise combined to form an island 107. Charcoal layer 104 may be positioned between backing layer 102 and island 107. In some embodiments, wound dressing 100 includes a removable cover sheet 103 to cover island 107 before use.

Backing Layer

Backing layer 102 is shown to include a first side 110 and a second, wound-facing side 112 opposite first side 110. When wound dressing 100 is applied to a wound, first side 110 faces away from the wound whereas second side 112 faces toward the wound. Backing layer 102 supports charcoal layer 104 and island 107 and provides a barrier to passage of microorganisms through wound dressing 100. In some embodiments, backing layer 102 is a thin layer of polyurethane film. One example of a suitable material for backing layer 102 is the polyurethane film known as ESTANE 5714F. Other suitable polymers for forming backing layer 102 include poly alkoxyalkyl acrylates and methacrylates, such as those described in Great Britain Patent Application No. 1280631A filed November 22, 2002. In some embodiments, backing layer 102 includes a continuous layer of a high-density blocked polyurethane foam that is predominantly closed-cell. Backing layer 102 may have a thickness in the range of 10 $\mu$m to 100 $\mu$m, preferably in the range of 50 $\mu$m to 70 $\mu$m. In some embodiments, backing layer 102 has a thickness of approximately 60 $\mu$m.

Backing layer 102 may be substantially impermeable to liquid and substantially permeable to moisture vapor. In other words, backing layer 102 may be permeable to water vapor, but not permeable to liquid water or wound exudate. This increases the total fluid handling capacity (TFHC) of wound dressing 100 while promoting a moist wound environment. In some embodiments, backing layer 102 is also impermeable to bacteria and other microorganisms. Backing layer 102 may have a moisture vapor transmission rate (MVTR) of approximately 300 to $5000 \frac{g}{m^2 \cdot 24\ hours}$, preferably 500 to $2000 \frac{g}{m^2 \cdot 24\ hours}$ at 37.5 °C at 100% to 10% relative humidity difference. In some embodiments, backing layer 102 is configured to wick moisture from charcoal layer 104 and distribute the moisture across first side 110.

Side 112 of backing layer 102 may be coated with an acrylic or other adhesive. The adhesive applied to side 112 ensures that wound dressing 100 adheres to surface 126 and that wound dressing 100 remains in place throughout the wear time. In some embodiments, the perimeter of backing layer 102 extends beyond (e.g., circumscribes) the perimeters of charcoal layer 104 and island 107 to provide an adhesive-coated margin for adhering wound dressing 100 to the skin of a patient adjacent to the wound being treated, shown in FIG. 4 as surface 126. The adhesive-coated margin may extend around all sides of charcoal layer 104 and island 107 such that wound dressing 100 is a so-called island dressing. In other embodiments, the adhesive-coated margin can be eliminated and wound dressing 100 can be adhered to surface 126 using other techniques.

In some embodiments, side 112 of backing layer 102 contacts side 114 of charcoal layer 104. Side 112 of backing layer 102 may be adhered to side 114 of charcoal layer 104 or may simply contact side 114 without the use of an adhesive. The perimeter of superabsorbent layer 106 may extend beyond (i.e., circumscribe) the perimeter of charcoal layer 104 to provide a margin around the perimeter of charcoal layer 104. Side 112 of backing layer 102 may extend beyond the perimeter of charcoal layer 104 to contact side 118 of superabsorbent layer 106. Side 112 of backing layer 102 may adhere to side 118 of superabsorbent layer 106 along the margin that extends beyond charcoal layer 104. In this way, backing layer 102 and superabsorbent layer 106 may form a closed pocket, sealing charcoal layer 104 between backing layer 102 and superabsorbent layer 106.

In some embodiments, the adhesive applied to side 112 of backing layer 102 is moisture vapor transmitting and/or patterned to allow passage of water vapor therethrough. The adhesive may include a continuous moisture vapor transmitting, pressure-sensitive adhesive layer of the type, conventionally used for island-type wound dressings (e.g., a polyurethane-based pressure sensitive adhesive). One example of an adhesive which can be used is a pressure sensitive adhesive based on acrylate ester copolymers, polyvinyl ethyl ether and polyurethane, as described in Great Britain Patent Application No. 1280631A. The basis weight of the adhesive may be 20 to 250 g/m$^2$, and more preferably 50 to 150 g/m$^2$.

Charcoal Layer

Charcoal layer 104 is shown to include a first side 114 and a second, wound-facing side 116 opposite first side 114. When wound dressing 100 is applied to a wound, first side 114 faces away from the wound whereas second side 116 faces toward the wound. In some embodiments, first side 114 of charcoal layer 104 contacts second side 112 of backing layer 102. Similarly, second side 116 of charcoal layer 104 may contact first side 118 of superabsorbent layer 106. Charcoal layer 104 may be adhered to backing layer 102 and superabsorbent layer 106 along surfaces 112, 114, 116, and/or 118. Alternatively, charcoal layer 104 may be sealed in a closed pocket between backing layer 102 and superabsorbent layer 106 such that adhesive is not required to fix charcoal layer 104 to backing layer 102 and/or superabsorbent layer 106.

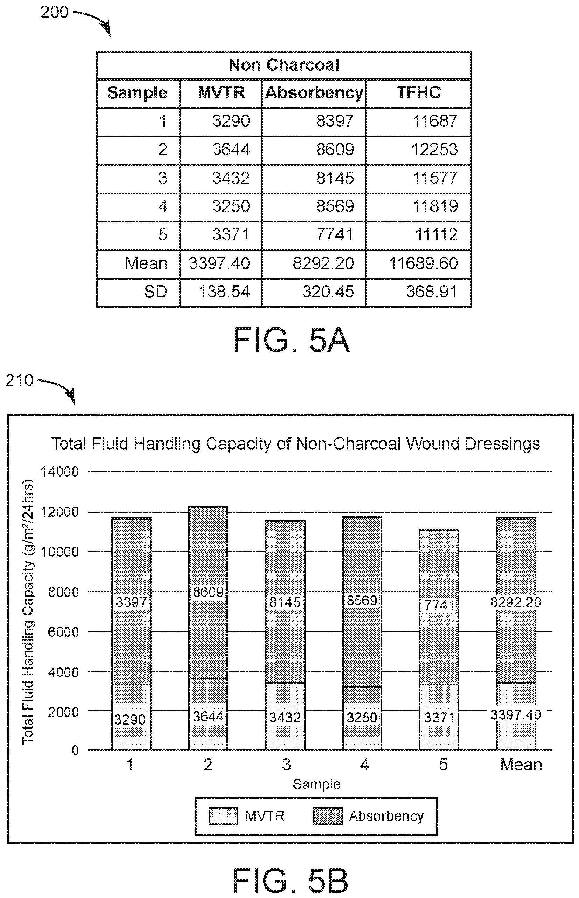

Advantageously, charcoal layer 104 may increase the MVTR, absorbency, and total fluid handling capacity (TFHC) of wound dressing 100 relative to similar wound dressings without charcoal layer 104. The increase in MVTR provided by charcoal layer 104 is unexpected given that adding another layer typically decreases MVTR. However, the unique combination of layers included in wound dressing 100 increases these properties while providing other benefits such as odor absorption. Several tables and graphs illustrating the results of testing performed to demonstrate this increase in MVTR, absorbency, and TFHC are shown in FIGS. 5A-7B, described in greater detail below.

Charcoal layer 104 may include activated charcoal (e.g., an activated charcoal cloth) having a plurality of small, low-volume pores within the internal volume of charcoal layer 104. The pores increase the internal surface area of charcoal layer 104 available for adsorption of the wound fluid. It is believed that the pores within charcoal layer 104 contribute to the increase in MVTR, absorbency, and TFHC. Small pores can be used to achieve a large internal surface area available for adsorption of the wound fluid, whereas larger pores may result in less surface area available for adsorption. Accordingly, it may be desirable to use relatively smaller pores to increase MVTR, absorbency, and TFHC. In some embodiments, charcoal layer 104 has a surface area available for adsorption in the range of 500 to 1500 m$^2$/g (i.e., per gram of the activated charcoal) and a pore volume in the range of 0.3 to 0.8 cm$^3$/g (i.e., per gram of the activated charcoal). However, it is contemplated that other surface areas and pore volumes can also be used to achieve a similar effect.

Another advantage provided by charcoal layer 104 is odor absorption. Charcoal layer 104 may have odor-absorbent properties and may function to reduce wound odor. The odor-absorbent properties of charcoal layer 104 may result from charcoal layer 104 trapping odor within the plurality of small, low-volume pores. The odor-absorbent properties of charcoal layer 104, in combination with the increase in MVTR, absorbency, and TFHC, provide a multifunctional wound dressing 100 that both manages wound exudate and reduces wound odor without requiring multiple wound dressings.

In some embodiments, charcoal layer 104 includes one or more therapeutic or antimicrobial agents. Therapeutic agents can include, for example, growth factors, analgesics, local anaesthetics and steroids. Antimicrobial agents can include antiseptics such as silver compounds (e.g. silver sulfadiazine) and chlorhexidine, and antibiotics. Several examples of therapeutic and antimicrobial agents which can be added to charcoal layer 104 are described in detail in U.S. Patent No. 8,962,908 issued February 24, 2015, U.S. Patent No. 8,858,987 issued October 14, 2014, and U.S. Patent No. 8,124,826 issued February 28, 2012.

Adding an antimicrobial agent to charcoal layer 104 may be desirable for several reasons. For example, combining the antimicrobial agent with charcoal layer 104 eliminates the need for a separate antimicrobial agent or layer, which simplifies manufacturing as well as wound treatment. Additionally, separating the antimicrobial agent from the wound may prevent unnecessary exposure to the antimicrobial when it is not needed, which can maintain the moisture level of the wound within a desirable range (e.g., drier wounds). In the presence of higher exudate, higher levels of the antimicrobial will be released as charcoal layer 104 becomes wet. This may cause the antimicrobial agent to migrate against the antimicrobial concentration gradient into the wound.

In some embodiments, charcoal layer 104 may be replaced with another intermediate layer between backing layer 102 and superabsorbent layer 106. The intermediate layer may include an activated charcoal cloth, an antimicrobial alginate cloth, a gauze, or a knitted yarn. These types of materials may also increase the MVTR, absorbency, and TFHC of wound dressing 100 relative to similar wound dressings without these additional layers.

In some embodiments, wound dressing 100 includes one or more liquid-permeable layers. A liquid-permeable layer may be located between charcoal layer 104 and backing layer 102 and/or between charcoal layer 104 and superabsorbent layer 106. For embodiments in which charcoal layer 104 is replaced with another intermediate layer, the liquid-permeable layers may be located between the intermediate layer and backing layer 102 and/or between the intermediate layer and superabsorbent layer 106. In some embodiments, wound dressing 100 includes a pair of liquid-permeable layers that envelop charcoal layer 104 or the intermediate layer (i.e., located on both sides of charcoal layer 104 or the intermediate layer).

In some embodiments, the liquid-permeable layers function to contain charcoal layer 104 and/or the intermediate layer. For example, charcoal layer 104 and/or the intermediate layer may be susceptible to breaking into several smaller pieces when wet. The liquid-permeable layers can be placed on opposite sides of charcoal layer 104 and/or the intermediate layer in order to reduce movement of the broken pieces within charcoal layer 104 and/or the intermediate layer.

In some embodiments, the liquid-permeable layers are made of a liquid-permeable fabric such as nylon. For example, the liquid-permeable layers may be made of a nylon fabric having a mass density of approximately 50 grams per square meter (GSM) of the fabric. The nylon fabric may be similar to or the same as the nylon fabric used in the ACTISORB brand wound dressing produced by Acelity. In some embodiments, the nylon fabric is spun-bonded and/or non-woven. The nylon fabric can be configured to allow liquid to permeate the fabric while keeping charcoal layer 104 and/or the intermediate layer contained between layers of the nylon fabric.

A benefit of using nylon non-woven fabric is that the fabric can be heated to bond to itself. For example, the layers of nylon fabric may be sized such that the perimeters of the nylon fabric layers extend beyond (e.g., circumscribe) the perimeter of charcoal layer 104 and/or the intermediate layer. The perimeters of the nylon fabric can be bonded to each other by applying heat to form a sealed pocket containing charcoal layer 104 and/or the intermediate layer between layers of the nylon fabric.

## Superabsorbent Layer

Superabsorbent layer 106 is shown to include a first side 118 and a second, wound-facing side 120 opposite first side 118. When wound dressing 100 is applied to a wound, first side 118 faces away from the wound whereas second side 120 faces toward the wound. In some embodiments, first side 118 of superabsorbent layer 106 contacts second side 116 of charcoal layer 104. Similarly, second side 120 of superabsorbent layer 106 may contact first side 122 of hydrophilic foam layer 108. In some embodiments, superabsorbent layer 106 is laminated to hydrophilic foam layer 108 using a fusible fiber positioned between superabsorbent layer 106 and hydrophilic foam layer 108. In some embodiments, superabsorbent layer 106 is configured to wick moisture from hydrophilic foam layer 108 and distribute the moisture across first side 118.

In some embodiments, superabsorbent layer 106 includes a hydrogel or hydrogel composition. Several examples of hydrogels and hydrogel compositions which can be used to form superabsorbent layer 106 are described in detail in U.S. Patent No. 8,097,272 issued January 17, 2012, U.S. Patent No. 8,664,464 issued March 4, 2014, and U.S. Patent No. 8,058,499 issued November 15, 2011.

The expressions "hydrogel" and "hydrogel compositions" used herein are not to be considered as limited to gels which contain water, but extend generally to all hydrophilic gels and gel compositions, including those containing organic non-polymeric components in the absence of water. For example, superabsorbent layer 106 may be formed from a polyurethane that entraps water to form a gel. In some embodiments, superabsorbent layer 106 is substantially continuous and/or substantially non-porous or non-foamed. Superabsorbent layer may include a flexible plasticized hydrophilic polymer matrix having a substantially continuous internal structure. The density of superabsorbent layer 106 may be greater than 0.5 g/cm$^3$, more preferably greater than 0.8 g/cm$^3$, and most preferably from 0.9 to 1.1 g/cm$^3$. In some embodiments, the thickness of superabsorbent layer 106 is from 1 mm to 10 mm, more preferably from 2 mm to 5 mm.

In some embodiments, superabsorbent layer 106 is cross-linked and preferably it is substantially insoluble in water at ambient temperature. However, the structure of superabsorbent layer 106 absorbs and entraps liquid to provide a highly hydrated gel structure in contrast to the porous foam structure of hydrophilic foam layer 108. Preferably, the gel can absorb 1 to 10 g/g of physiological saline at 20°, more preferably 2 to 5 g/g.

In some embodiments, the dry weight of superabsorbent layer 106 is from 1000 to 5000 g/m$^2$, more preferably from 2000 to 4000 g/m$^2$. In some embodiments, superabsorbent layer 106 includes from 1% to 30% of water, more preferably from 10% to 20% by weight of water before use. In some embodiments, superabsorbent layer 106 contains from 1% to 40%, more preferably from 5 to 15%, by weight of one or more humectants, preferably selected from the group consisting of glycerol, propylene glycol, sorbitol, mannitol, polydextrose, sodium pyrrolidine carboxylic acid (NaPCA), hyaluronic acid, aloe, jojoba, lactic acid, urea, gelatin, lecithin and mixtures thereof. The entrapped water and optional humectants give the hydrogel a soft, moist wound-friendly surface for contacting the wound.

Hydrophilic Foam Layer

Hydrophilic foam layer 108 is shown to include a first side 122 and a second, wound-facing side 124 opposite first side 122. When wound dressing 100 is applied to a wound, first side 122 faces away from the wound whereas second side 124 faces toward the wound. In some embodiments, first side 122 of hydrophilic foam layer 108 contacts second side 120 of superabsorbent layer 106.

In some embodiments, hydrophilic foam layer 108 is laminated to superabsorbent layer 106 using a fusible fiber positioned between superabsorbent layer 106 and hydrophilic foam layer 108. For example, superabsorbent layer 106 may be applied to first side 122 of hydrophilic foam layer 108 and may at least partially cover first side 122. Superabsorbent layer 106 can be bonded to hydrophilic foam layer 108, for example by an adhesive or by radiation cross-linking. In some embodiments, superabsorbent layer 106 is bonded to the hydrophilic foam layer 108 by urethane or urea linkages. This can be achieved by applying hydrophilic foam layer 108 to superabsorbent layer 106 (substantially without mixing) before polyurethane curing is complete.

Hydrophilic foam layer 108 may include a polyurethane foam coupled to side 120 of superabsorbent layer 106. In some embodiments, hydrophilic foam layer 108 includes a flexible plasticized hydrophilic polymer matrix having an internal cellular structure. Several examples of hydrophilic foams which can be used to form hydrophilic foam layer 108 are described in detail in U.S. Patent No. 8,097,272 issued January 17, 2012, U.S. Patent No. 8,664,464 issued March 4, 2014, and U.S. Patent No. 8,058,499 issued November 15, 2011.

Advantageously, hydrophilic foam layer 108 provides enhanced absorbency for liquid exudate. This is because the initial substantially anhydrous condition and porous structure of hydrophilic foam layer 108 enables it to absorb a larger amount of water by both chemical and physical absorption that is the case for the corresponding hydrogel material. Furthermore, the porous structure of the foam provides for rapid uptake of liquid exudate, in contrast to pure hydrogel dressings.

In some embodiments, hydrophilic foam layer 108 has a thickness of from 1 to 20 mm, more preferably from 1.5 to 5 mm In some embodiments, hydrophilic foam layer 108 has a density of from 0.28 g/cm³ to 0.5 g/cm³, and more preferably from 0.32 g/cm³ to 0.48 g/cm³. Preferably, hydrophilic foam layer 108 has an elongation to break of at least 150%, more preferably from 500% to 1000%. The foam that forms layer 108 may be hydrophilic and can absorb aqueous fluids such as wound exudate with swelling. Hydrophilic foam layer 108 may be highly cross-linked and substantially insoluble in water.

In some embodiments, hydrophilic foam layer 108 has an absorbency of at least 3 grams of saline per gram of foam, and preferably a swellability in water of at least 200%. In some embodiments, hydrophilic foam layer 108 is constructed using the foam as described in European Patent No. 0541391 issued June 10, 1998. In some embodiments, hydrophilic foam layer 108 includes less than 10% water prior to use as an absorbent, more preferably less than 5% water, and even more preferably it contains less than 2% of water before use.

**Test Results**

Referring now to FIGS. 5A-7B, several tables and graphs illustrating the performance of wound dressing 100 relative to similar wound dressings without charcoal layer 104 are shown, according to an exemplary embodiment. FIG. 5A is a data table 200 indicating the moisture vapor transmission rate (MVTR), absorbency, and total fluid handling capacity (TFHC) of several samples of wound dressings without charcoal layer 104. MVTR, absorbency, and TFHC are measured in units of $\frac{g}{m^2 \cdot 24\ hours}$. The TFHC is defined as the sum of MVTR and absorbency (i.e., $MVTR + absorbency = TFHC$). FIG. 5B is a column graph 210 plotting the test results shown in data table 200. As shown in FIGS. 5A-5B, the wound dressing samples without charcoal layer 104 have a mean MVTR of $3,397.4\ \frac{g}{m^2 \cdot 24\ hours}$, a mean absorbency of $8,292.2\ \frac{g}{m^2 \cdot 24\ hours}$, and a mean TFHC of $11,689.6\ \frac{g}{m^2 \cdot 24\ hours}$.

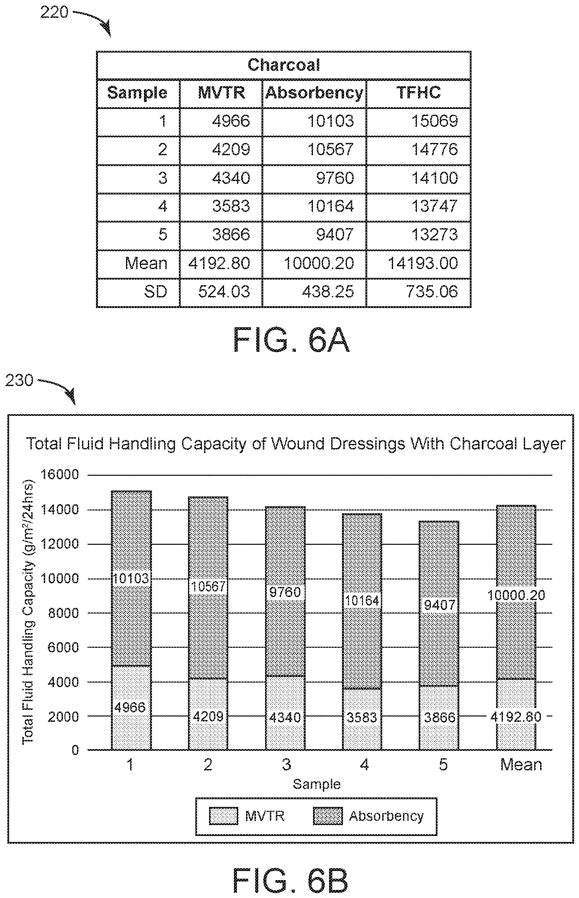

FIG. 6A is a data table 220 indicating the MVTR, absorbency, and TFHC of several samples of wound dressing 100 with charcoal layer 104. FIG. 6B is a column graph 230 plotting the test results shown in data table 220. As shown in FIGS. 6A-6B, the samples of wound dressing 100 with charcoal layer 104 have a mean MVTR of $4,192.8\ \frac{g}{m^2 \cdot 24\ hours}$, a mean absorbency of $10,000.2\ \frac{g}{m^2 \cdot 24\ hours}$, and a mean TFHC of $14,193.0\ \frac{g}{m^2 \cdot 24\ hours}$.

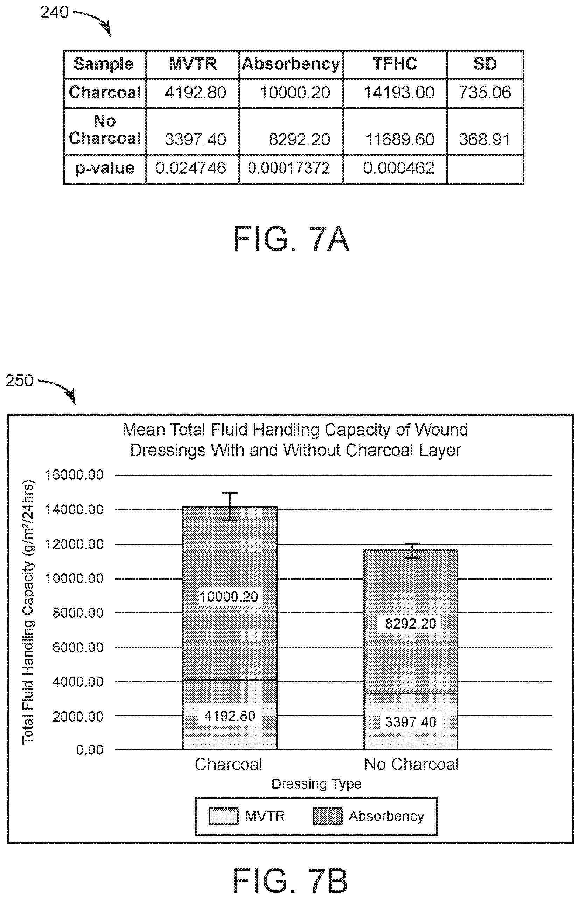

FIG. 7A is a data table 240 summarizing the mean test results shown in data tables 200 and 220. FIG. 7B is a column graph 250 plotting the test results shown in data table 240. The addition of charcoal layer 104 to wound dressing 100 increased MVTR by 795.4 $\frac{g}{m^2 \cdot 24\ hours}$, which is over 23% of the MVTR value without charcoal layer 104. The addition of charcoal layer 104 also increased absorbency by 1,708.0 $\frac{g}{m^2 \cdot 24\ hours}$, which is over 20% of the absorbency value without charcoal layer 104. Consequently, the addition of charcoal layer 104 increased TFHC by 2,503.4 $\frac{g}{m^2 \cdot 24\ hours}$, which is over 21% or the TFHC value without charcoal layer 104.

A statistical hypothesis test (e.g., a t-test) was performed on these test results to determine whether the increases in MVTR, absorbency, and TFHC are statistically significant. The statistical hypothesis test showed that the samples of wound dressing 100 with charcoal layer 104 have a significantly higher MVTR, absorbency, and TFHC relative to the samples without charcoal layer 104. This indicates that the samples of wound dressing 100 with charcoal layer 104 are significantly better than the samples without charcoal layer 104 at absorbing and transmitting moisture from the wound.

It is contemplated that charcoal layer 104 could be used to increase the MVTR of a sample with a thick backing layer 102. As the thickness of a backing layer 102 increases, the MVTR decreases. If a higher MVTR was desired but the thickness of backing layer 102 could not be altered; the addition of charcoal layer 102 could be beneficial. As well as increasing MVTR, absorbency, and TFHC compared to the non-charcoal samples, charcoal layer 104 can also act as an odor absorber. This would be beneficial to patients' quality of life. An antimicrobial compound (e.g., silver) can be added to charcoal layer 104 to combat bacteria in wounds.

**Configuration of Exemplary Embodiments**

The construction and arrangement of the systems and methods as shown in the various exemplary embodiments are illustrative only.

**Claims**

1. A wound dressing (100) comprising:

   a superabsorbent layer (106) configured to absorb wound fluid and having a first side and a second, wound-facing side;
   a backing layer (102) having a first side and a second, wound-facing side, wherein the backing layer (102) is substantially impermeable to liquid and substantially permeable to vapor; and
   a charcoal layer (104) configured to increase a moisture vapor transmission rate of the wound dressing (100), wherein the charcoal layer (104) comprises activated charcoal having a plurality of pores that increase a surface area available for adsorption of the wound fluid,
   the charcoal layer (104) comprising:

      a first side positioned adjacent to and abutting the second, wound-facing side of the backing layer (102); and
      a second, wound-facing side positioned adjacent to and abutting the first side of the superabsorbent layer (106).

2. The wound dressing (100) of Claim 1, wherein the surface area available for adsorption of the wound fluid is in the range of 500 to 1500 m² per gram of the activated charcoal.

3. The wound dressing (100) of Claim 1, wherein the pores of the activated charcoal have a volume in the range of 0.3 to 0.8 cm³ per gram of the activated charcoal.

4. The wound dressing (100) of Claim 1, wherein the charcoal comprises antimicrobial silver.

5. The wound dressing (100) of Claim 1, further comprising two or more liquid-permeable layers enveloping the charcoal layer (104), the liquid-permeable layers configured to contain the charcoal layer (104) and reduce movement of the charcoal layer (104).

6. The wound dressing (100) of Claim 5, wherein one of the liquid-permeable layers is located between the backing layer (102) and the charcoal layer (104) and another of the liquid-permeable layers is located between the superabsorbent layer (106) and the charcoal layer (104).

7. The wound dressing (100) of Claim 5, wherein the liquid-permeable layers are made of a nylon fabric comprising at least one of a spun-bonded nylon and a non-woven nylon.

8. The wound dressing (100) of Claim 1, further comprising a hydrophilic polyurethane foam positioned adjacent to and abutting the second, wound-facing side of the superabsorbent layer (106).

9. The wound dressing (100) of Claim 8, wherein the superabsorbent layer (106) is laminated with fusible fiber to the hydrophilic polyurethane foam.

10. The wound dressing (100) of Claim 8, wherein:

    the hydrophilic polyurethane foam comprises a flexible plasticized hydrophilic polymer matrix having an internal cellular structure; and

the superabsorbent layer (106) comprises a flexible plasticized hydrophilic polymer matrix having a substantially continuous internal structure.

11. The wound dressing (100) of Claim 8, wherein the superabsorbent layer (106) is configured to wick moisture from the hydrophilic polyurethane foam and distribute the moisture across the first side of the superabsorbent layer (106).

12. The wound dressing (100) of Claim 1, wherein the backing layer (102) comprises a polyurethane film.

13. The wound dressing (100) of Claim 1, wherein the backing layer (102) is configured to wick moisture from the charcoal layer (104) and distribute the moisture across the first side of the backing layer (102).

14. The wound dressing (100) of Claim 1, further comprising an adhesive coating coupled to the second, wound-facing side of the backing layer (102);

optionally wherein the backing layer (102) extends beyond a perimeter of the charcoal layer (104) and beyond a perimeter of the superabsorbent layer (106) to provide an adhesive-coated margin configured to adhere the wound dressing (100) to a surface;
optionally wherein a perimeter of the superabsorbent layer (106) extends beyond a perimeter of the charcoal layer (104); and the backing layer (102) is adhered to both the charcoal layer (104) and the perimeter of the superabsorbent layer (106) via the adhesive coating, thereby sealing the charcoal layer (104) between the backing layer and the superabsorbent layer (106).

15. The wound dressing (100) of Claim 1, further comprising at least one removable cover sheet (103) to cover the second, wound-facing side of the superabsorbent layer (106) before use.

**Patentansprüche**

1. Ein Wundverband (100), aufweisend:

eine Superabsorptionsschicht (106), die konfiguriert ist, um Wundflüssigkeit zu absorbieren, und die eine erste Seite und eine zweite, der Wunde zugewandte Seite aufweist;
eine Trägerschicht (102), die eine erste Seite und eine zweite, der Wunde zugewandte Seite aufweist, wobei die Trägerschicht (102) im Wesentlichen flüssigkeitsundurchlässig und im Wesentlichen dampfdurchlässig ist; und
eine Kohleschicht (104), die konfiguriert ist, um eine Feuchtigkeitsdampfdurchlässigkeitsrate des Wundverbands (100) zu erhöhen, wobei die Kohleschicht (104) Aktivkohle umfasst, die eine Mehrzahl von Poren aufweist, die einen zur Adsorption der Wundflüssigkeit verfügbaren Oberflächenbereich vergrößern,
die Kohleschicht (104) aufweisend:

eine erste Seite, die neben der zweiten, der Wunde zugewandten Seite der Trägerschicht (102) positioniert ist und an diese angrenzt; und
eine zweite, der Wunde zugewandte Seite, die neben der ersten Seite der Superabsorbtionsschicht (106) positioniert ist und an diese angrenzt.

2. Der Wundverband (100) nach Anspruch 1, wobei der zur Adsorption der Wundflüssigkeit verfügbare Oberflächenbereich im Bereich von 500 bis 1500 m² pro Gramm der Aktivkohle liegt.

3. Der Wundverband (100) nach Anspruch 1, wobei die Poren der Aktivkohle ein Volumen im Bereich von 0,3 bis 0,8 cm³ pro Gramm der Aktivkohle aufweisen.

4. Der Wundverband (100) nach Anspruch 1, wobei die Aktivkohle antimikrobielles Silber aufweist.

5. Der Wundverband (100) nach Anspruch 1, ferner aufweisend zwei oder mehrere flüssigkeitsdurchlässige Schichten, die die Kohleschicht (104) umhüllen, wobei die flüssigkeitsdurchlässigen Schichten konfiguriert sind, um die Kohleschicht (104) zu enthalten und eine Bewegung der Kohleschicht (104) zu verringern.

6. Der Wundverband (100) nach Anspruch 5, wobei sich eine der flüssigkeitsdurchlässigen Schichten zwischen der Trägerschicht (102) und der Kohleschicht (104) befindet und sich eine andere der flüssigkeitsdurchlässigen Schichten zwischen der Superabsorbtionsschicht (106) und der Kohleschicht (104) befindet.

7. Der Wundverband (100) nach Anspruch 5, wobei die flüssigkeitsdurchlässigen Schichten aus einem Nylon-Gewebe hergestellt sind, das mindestens eines von einem gesponnenen Nylon und einem Nylonvlies aufweist.

8. Der Wundverband (100) nach Anspruch 1, ferner umfassend einen hydrophilen Polyurethanschaum, der neben der zweiten, der Wunde zugewandten Seite der Superabsorbtionsschicht (106) positioniert ist und an diese angrenzt.

9. Der Wundverband (100) nach Anspruch 8, wobei die Superabsorbtionsschicht (106) an den hydrophilen Polyurethanschaum mit schmelzbarer Faser laminiert ist.

10. Der Wundverband (100) nach Anspruch 8, wobei:

der hydrophile Polyurethanschaum eine flexible plastifizierte hydrophile Polymermatrix aufweist, die eine innere Zellstruktur aufweist; und
die Superabsorbtionsschicht (106) eine flexible plastifizierte hydrophile Polymermatrix aufweist, die eine im Wesentlichen kontinuierliche innere Struktur aufweist.

11. Der Wundverband (100) nach Anspruch 8, wobei die Superabsorbtionsschicht (106) konfiguriert ist, um Feuchtigkeit aus dem hydrophilen Polyurethanschaum zu leiten und die Feuchtigkeit über die erste Seite der Superabsorbtionsschicht (106) zu verteilen.

12. Der Wundverband (100) nach Anspruch 1, wobei die Trägerschicht (102) eine Polyurethanfolie aufweist.

13. Der Wundverband (100) nach Anspruch 1, wobei die Trägerschicht (102) konfiguriert ist, um Feuchtigkeit aus der Kohleschicht (104) zu leiten und die Feuchtigkeit über die erste Seite der Trägerschicht (102) zu verteilen.

14. Der Wundverband (100) nach Anspruch 1, ferner aufweisend eine Haftmittelbeschichtung, die mit der zweiten, der Wunde zugewandten Seite der Trägerschicht (102) gekoppelt ist;

wobei sich wahlweise die Trägerschicht (102) über einen Umfang der Kohleschicht (104) hinaus und über einen Umfang der Superabsorbtionsschicht (106) hinaus erstreckt, um eine haftmittelbeschichtete Marge bereitzustellen, die konfiguriert ist, um den Wundverband (100) an eine Oberfläche anzuhaften;
wobei sich wahlweise ein Umfang der Superabsorbtionsschicht (106) über einen Umfang der Kohleschicht (104) hinaus erstreckt; und die Trägerschicht (102) über die Haftmittelbeschichtung sowohl an der Kohleschicht (104) als auch an dem Umfang der Superabsorbtionsschicht (106) angehaftet ist, wodurch die Kohleschicht (104) zwischen der Trägerschicht und der Superabsorbtionsschicht (106) abgedichtet wird.

15. Der Wundverband (100) nach Anspruch 1, ferner aufweisend mindestens eine entfernbare Abdecklage (103) zur Abdeckung der zweiten, der Wunde zugewandten Seite der Superabsorbtionsschicht (106) vor der Verwendung.

**Revendications**

1. Pansement pour plaie (100) comprenant :

une couche superabsorbante (106) configurée pour absorber un fluide de plaie et ayant un premier côté et un deuxième côté faisant face à la plaie ;
une couche de support (102) ayant un premier côté et un deuxième côté faisant face à la plaie, dans lequel la couche de support (102) est sensiblement imperméable à un liquide et sensiblement perméable à la vapeur ; et
une couche de charbon de bois (104) configurée pour augmenter un taux de transmission de vapeur humide du pansement pour plaie (100), dans lequel la couche de charbon de bois (104) comprend du charbon de bois actif ayant une pluralité de pores qui augmentent la superficie disponible pour adsorption du fluide de plaie, la couche de charbon de bois (104) comprenant :

un premier côté positionné adjacent à et venant en butée contre le deuxième côté faisant face à la plaie de la couche de support (102) ; et
un deuxième côté faisant face à la plaie positionné adjacent à et venant en butée contre le premier côté de la couche superabsorbante (106).

2. Pansement pour plaie (100) selon la revendication 1, dans lequel la superficie disponible pour adsorption du fluide de plaie est dans la plage de 500 à 1500 m$^2$ par gramme du charbon de bois actif.

3. Pansement pour plaie (100) selon la revendication 1, dans lequel les pores du charbon de bois actif ont un volume dans la plage de 0,3 à 0,8 cm$^3$ par gramme du charbon de bois actif.

4. Pansement pour plaie (100) selon la revendication 1, dans lequel le charbon de bois comprend de l'argent antimicrobien.

5. Pansement pour plaie (100) selon la revendication 1, comprenant en outre deux couches perméables aux liquides ou plus enveloppant la couche de charbon de bois (104), les couches perméables aux liquides configurées pour contenir la couche de charbon de bois (104) et réduire un mouvement de la couche de charbon de bois (104).

6. Pansement pour plaie (100) selon la revendication 5, dans lequel une des couches perméables aux liquides se situe entre la couche de support (102) et la couche de charbon de bois (104) et une autre des couches perméables aux liquides se situe entre la couche superabsorbante (106) et la couche de charbon de bois (104).

7. Pansement pour plaie (100) selon la revendication 5, dans lequel les couches perméables aux liquides sont constituées d'un tissu de nylon comprenant au moins un parmi un nylon filé-lié et un nylon non tissé.

8. Pansement pour plaie (100) selon la revendication 1, comprenant en outre une mousse de polyuréthane hydrophile positionnée adjacente à et venant en butée contre le deuxième côté faisant face à la plaie de la couche superabsorbante (106).

9. Pansement pour plaie (100) selon la revendication 8, dans lequel la couche superabsorbante (106) est stratifiée avec une fibre fusible à la mousse de polyuréthane hydrophile.

10. Pansement pour plaie (100) selon la revendication 8, dans lequel :

la mousse de polyuréthane hydrophile comprend une matrice polymère hydrophile plastifiée flexible ayant une structure alvéolaire interne ; et
la couche superabsorbante (106) comprend une matrice polymère hydrophile plastifiée flexible ayant une structure interne sensiblement continue.

11. Pansement pour plaie (100) selon la revendication 8, dans lequel la couche superabsorbante (106) est configurée pour drainer par capillarité l'humidité provenant de la mousse de polyuréthane hydrophile et répartir l'humidité à travers le premier côté de la couche superabsorbante (106).

12. Pansement pour plaie (100) selon la revendication 1, dans lequel la couche de support (102) comprend un film de polyuréthane.

13. Pansement pour plaie (100) selon la revendication 1, dans lequel la couche de support (102) est configurée pour drainer par capillarité l'humidité provenant de la couche de charbon de bois (104) et répartir l'humidité à travers le premier côté de la couche de support (102).

14. Pansement pour plaie (100) selon la revendication 1, comprenant en outre un revêtement adhésif couplé au deuxième côté faisant face à la plaie de la couche de support (102) ;

facultativement dans lequel la couche de support (102) s'étend au-delà d'un périmètre de la couche de charbon de bois (104) et au-delà d'un périmètre de la couche superabsorbante (106) pour fournir une marge revêtue d'adhésif configurée pour faire adhérer le pansement pour plaie (100) à une surface ;
facultativement dans lequel un périmètre de la couche superabsorbante (106) s'étend au-delà d'un périmètre de la couche de charbon de bois (104) ; et la couche de support (102) est fixée par adhérence à la fois à la couche de charbon de bois (104) et au périmètre de la couche superabsorbante (106) par l'intermédiaire du revêtement adhésif, ce qui scelle la couche de charbon de bois (104) entre la couche de support et la couche superabsorbante (106).

15. Pansement pour plaie (100) selon la revendication 1, comprenant en outre au moins une feuille de couverture amovible (103) pour couvrir le deuxième côté faisant face à la plaie de la couche superabsorbante (106) avant utilisation.

FIG. 1

FIG. 2

FIG. 3

FIG. 4

200

| Non Charcoal | | | |
|---|---|---|---|
| Sample | MVTR | Absorbency | TFHC |
| 1 | 3290 | 8397 | 11687 |
| 2 | 3644 | 8609 | 12253 |
| 3 | 3432 | 8145 | 11577 |
| 4 | 3250 | 8569 | 11819 |
| 5 | 3371 | 7741 | 11112 |
| Mean | 3397.40 | 8292.20 | 11689.60 |
| SD | 138.54 | 320.45 | 368.91 |

## FIG. 5A

210

FIG. 5B

220

| Charcoal | | | |
|---|---|---|---|
| Sample | MVTR | Absorbency | TFHC |
| 1 | 4966 | 10103 | 15069 |
| 2 | 4209 | 10567 | 14776 |
| 3 | 4340 | 9760 | 14100 |
| 4 | 3583 | 10164 | 13747 |
| 5 | 3866 | 9407 | 13273 |
| Mean | 4192.80 | 10000.20 | 14193.00 |
| SD | 524.03 | 438.25 | 735.06 |

## FIG. 6A

230

FIG. 6B

240 ⌐

| Sample | MVTR | Absorbency | TFHC | SD |
|---|---|---|---|---|
| Charcoal | 4192.80 | 10000.20 | 14193.00 | 735.06 |
| No Charcoal | 3397.40 | 8292.20 | 11689.60 | 368.91 |
| p-value | 0.024746 | 0.00017372 | 0.000462 | |

# FIG. 7A

250 ⌐

FIG. 7B

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013007973 A2
- GB 2531345 A
- WO 8605970 A
- GB 1280631 A
- US 8962908 B
- US 8858987 B
- US 8124826 B
- US 8097272 B
- US 8664464 B
- US 8058499 B
- EP 0541391 A